# EUROPEAN PATENT APPLICATION

(11) **EP 0 528 532 A1**
(43) Date of publication of application: **24.02.1993**
(21) Application number: 92306369.7
(22) Date of filing: 10.07.1992
(51) Int. Cl.: A61K 31/71

(54) **Use of natamycin for the treatment and prevention of poultry mycosis**

(30) Priority: 12.07.1991 US 729375
(71) Applicant: DUCOA, Highland, Illinois 62249 (US)
(72) Inventor: King, Bruce Dexter, Troy, Illinois 62294 (US)
(74) Representative: Woodcraft, David Charles

(57) **Abstract**

Diseases associated with *Candida albican* yeast are controlled and prevented by including an effective amount of natamycin in the drinking water and/or feed of poultry.

## Description

### FIELD OF THE INVENTION

The present invention relates to a method for controlling and preventing diseases in poultry, and, more particularly, to treating crop mycosis in poultry caused by *Candida albicans* yeast with natamycin.

### BACKGROUND OF THE INVENTION

A species of yeast is the causative agent for an infectious disease of poultry known as crop mycosis. Crop mycosis is a yeast infection of the mucous walls of the crop. The disease affects primarily broilers, laying hens and turkeys. It is believed that in severe cases the disease may also infect the intestinal tract. In environments contaminated with yeast, poultry losses are indicated by poor growth and decreased egg production.

Natamycin is a member of the polyene family of antimycotics. The compound natamycin is a tetraene with a molecular weight of about 666, empirical formula corresponding generally to C₃₃H₄₇NO₁₃, and contains a glycosidically-linked carbohydrate moiety, mycosamine. It has an isoelectric point of pH 6.5. The structure of natamycin typically exists in two configurations: the enol-structure and the keto-structure.

Natamycin is known as an antibiotic in humans which is discussed in Florey, "Analytical Profiles of Drug Substances", Vol. 10, 1981. As reported in "Pharmacology", Natamycin by Wolfgang P. Raab, 1972, Georg Thieme Publishers, Stuttgart, pages 18 & 19, certain yeasts exhibit susceptibility to natamycin. Natamycin has also been used to enhance the effectiveness of lactulose in curing human vaginitis caused by *Candida albicans;* in the absence of lactulose, natamycin has only temporary effectiveness against human vaginities (Wooton U.S. Patent No. 3,860,707). The disclosure of each of the above publications is hereby incorporated by reference.

It is an object of the present invention to use natamycin for controlling and preventing diseases in poultry due to the presence of yeast which are present in the environment used for raising poultry. Particularly, the invention is directed to controlling and preventing diseases associated with *Candida albicans* yeast. The environmental characteristics of the sites on humans and poultry, which may be infected by *Candida albicans,* are diverse and, accordingly, the effectiveness of natamycin against a poultry infection of *Candida albicans* was a surprising discovery. The surprising effectiveness of natamycin is emphasized when considering the anatomical, metabolic, etc., differences between humans and poultry. It is another object of the present invention, to control and prevent a disease caused by an infection of *Candida albicans* by including natamycin in the feed and/or drinking water of the poultry.

Although particular emphasis is placed upon treating *Candida albicans* which affects poultry, other avians (e.g., ducks, turkeys, pheasants, etc.), may be effectively treated in accordance with the present invention. In addition, the invention may be used to control and prevent diseases associated with yeasts which are related to *Candida albicans*.

### SUMMARY OF THE INVENTION

The present invention relates to a method for controlling and preventing diseases in poultry associated with the *Candida albicans* yeast. This infection can be controlled and prevented and by treating the poultry exposed to *Candida albicans* with an effective amount of natamycin.

In one aspect of the invention, crop mycosis caused by an infection of *Candida albicans* may be treated by exposing the crop to natamycin. In accordance with this aspect of the invention, the natamycin can be applied to the crop as a solution or stable suspension in any suitable non-toxic carrier liquid. One particular technique for applying the natamycin to the crop is as a suspension in the poultry's drinking water (e.g., a stabilized aqueous suspension within a non-toxic carrier liquid). Concentrations of natamycin in the range of about 1 to about 70 parts per million (ppm) of the carrier (e.g., feed, water, etc.), are normally effective.

In another aspect of the invention, the presence of natamycin in the feed for poultry reduces yeast growth which may prevent the poultry from being infected. Further, the feed may function as the carrier of the natamycin to the poultry crop and, accordingly, control a disease caused by an infection of *Candida albicans*(e.g.,crop mycosis).

Moreover, early treatment with natamycin can prevent a yeast infection. Therefore, it is desirable to treat newly hatched chicks or poults with natamycin which may be followed by periodic treatment throughout the life of the poultry.

### DETAILED DESCRIPTION OF THE INVENTION

The method of the present invention relates to control and prevention of diseases associated with *Candida albicans* yeast. Particularly, the invention relates to control and prevention of diseases caused by an infection of *Candida albicans* by inhibiting, if not eliminating, growth of this undesired yeast. The undesired yeast comprising *Candida albicans*(hereinafter referred to as *Candida*), which may grow within and upon the feed that is consumed by the poultry. The growth and metabolic activities of the yeast, may in some cases, produce toxins which may be poisonous to the poultry. Moreover importantly, the undesired yeast may invade the bodies of the poultry to create diseases (e.g., crop mycosis), which are contagious and normally fatal.

In one embodiment of the invention, control and prevention of a disease associated with the undesired yeast is achieved by exposing the drinking water, feed and/or the poultry to dosage of natamycin which is effective to control *Candida* in the carrier (e.g., feed, water, etc.). An effective dosage of natamycin typically ranges from about 1.0 parts per million (ppm) through about 70 ppm concentration in the carrier and normally about 10 ppm. However, the exact dosage which is sufficient to be effective against the *Candida albicans* in the poultry, is dependent upon the Specific environment through which this undesired yeast is introduced into the poultry. In accordance with the invention, the term "environment" is intended to refer to the feed, water, infection site of the poultry, etc., which may be adversely affected by the *Candida* For example, if the environment is favorable for the metabolic activities of the yeast, a relatively large dosage of natamycin may be required to be effective against the yeast (e.g., 20-30 ppm of concentration of natamycin). Particularly, an environment which is warm, moist, possesses a neutral pH, contains oxygen, includes a substrate which enhances yeast growth, etc., would require a relatively large dosage of natamycin to be effective against the undesired yeast. Further, the amount of natamycin which is necessary to prevent growth or infection of the undesired yeast is less than the amount of natamycin which is necessary to treat an existing yeast infection. Therefore, it is advantageous to treat the poultry in accordance with the invention while the chicks or poults are in the early stages of their development (e.g., treat the chicks immediately after hatching to prevent an infection of yeast).

Moreover, although it is preferred to practice the invention by using natamycin, pharmaceutically acceptable derivatives of natamycin (e.g., calcium and sodium salts, and esters of natamycin), which are effective to control and prevent exposure of the poultry to *Candida albicans*, may be used in the invention alone or in conjunction with natamycin. The preferred morphology of natamycin is crystalline; however, any morphological state is acceptable in practice of the invention which is effective against *Candida.* Further, the natamycin used in the invention may be present in one or more hydrate forms (e.g., mono-, di- and tri-hydrates). The tri-hydrate form is advantageous for some applications due to its stability. In some aspects of the invention, it may be desirable to increase the effectiveness of the natamycin by comminuting or grinding the natamycin to increase the surface area of the natamycin.

In one embodiment of the invention, the drinking water which is consumed by the poultry is treated with natamycin. *Candida* may exist in water and thus, the natamycin may prevent an infection of the poultry via drinking water. Further, the drinking water which has been treated with natamycin may be used to treat an existing infection of *Candida* in the poultry. In this embodiment a stable suspension including natamycin may be added to the drinking water. The suspension may comprise natamycin and a non-toxic carrier liquid. Further, the non-toxic carrier should not be a solvent for natamycin. However, certain pharmaceutically acceptable derivatives of natamycin may be soluble within the non-toxic carrier liquid. Suitable non-toxic carrier liquids comprise at least one of the following group: alcohols such as methanol, water. etc. Moreover, in some aspects of this embodiment the natamycin may be added directly to the drinking water.

In a second embodiment of the invention, the natamycin may be used to treat the feed which is consumed by the poultry. Particularly, the natamycin may be added to the individual constituents of the feed which are normally crushed or ground prior to being fed to the poultry. Feed for poultry typically comprises at least one member of the following group: cereal grains (e.g., corn), soybeans, oil seeds, etc. In one aspect of this embodiment the natamycin is added to the feed after the constituents of the feed have been crushed or ground to an appropriate reduced size. Further, the natamycin may be admixed with one or more of the non-toxic carrier liquids, sprayed onto the poultry feed, etc., or applied in any manner which is adequate to carry or provide an effective quantity of natamycin to the poultry. The natamycin which is present in the feed can be used to reduce, if not prevent, growth of the undesired yeast and, accordingly, is normally effective as a treatment of an existing yeast infection (e.g., crop mycosis).

The ability of the natamycin to treat an existing infection within the poultry is due in part to the unique anatomy of poultry. For example, the orientation of the crop of a chicken is such that any feed or water consumed tends to flow past and contact the crop. Therefore, when an effective amount of natamycin is present in the feed and/or drinking water, the same may contact the crop in a manner which is adequate to be effective against a yeast infection (e.g., crop mycosis).

While not wishing to be bound by any theory or explanation, it is believed that natamycin is active or effective against growth of yeasts, but not against bacteria. One explanation may be that yeasts(but not bacteria) contain ergosterol in their membranes.

In general the yeast will grow, if the environment is appropriate, until contacting the natamycin. The mechanism of natamycin action suggested is a binding of the natamycin molecule and ergosterol present in the cell membrane of the yeast. The binding or complexing with ergosterol is substantiated by the neutralizing effects of ergosterol addition on the antifungal activities of natamycin against the *Candida albicans*. A complex between natamycin and the cell membrane of the yeast is believed to alter membrane permeability since natamycin is a relatively large molecule which creates an increased surface pressure which may tend to induce a reorientation of the ergosterol present in the membrane; thus altering permeability of the cell and resulting in osmotic shock. This osmotic shock is sufficient to interrupt, if not halt, the metabolic activities of the *Candida*(e.g., the natamycin may cause irregular yeast growth, discoloration, etc.)

Moreover, in one aspect of the invention, an energy source such as sugar (e.g., lactose) may be admixed with the natamycin which is added to the feed and/or drinking water. Particularly, without wishing to be bound by any theory or discussion, it is believed that the energy source may enhance the mechanism, discussed above, through which the natamycin is believed to be effective against the undesired yeast. Alternatively, the energy source may increase the ability of the poultry to combat an infection of *Candida*. The energy source may be either soluble or insoluble in the non-toxic liquid carrier.

In addition to the energy source, other materials may be used in conjunction with the natamycin. Particularly, there are certain readily available materials which may tend to reduce growth of the undesired yeast. For example, fish meal, vitamins, etc. may be added to the feed which is consumed by the poultry that may adversely affect the environment of the undesired yeast (e.g., the growth rate of the yeast is reduced). However, the presence of, for example, fish meal alone is not sufficient to reduce adequately the quantity of the undesired yeast. Therefore, in certain aspects of the invention, a combination of natamycin with other materials is effective against the undesired yeast.

Natamycin is also compatible with additives which may be admixed with the poultry feed and/or drinking water. Suitable additives include at least one member from the following group: protein fat, fiber, calcium (e.g., calcium carbonate), phosphorus, trace metals, vitamins, citrus pulp, grape hulls, rice hulls, etc.

The natamycin which has been added to the drinking water and/or feed of the poultry typically possesses an acceptable shelf-life. Depending upon the storage conditions, natamycin can be expected to be effective against the undesired yeasts for several weeks or months. However, to ensure the effectiveness of the natamycin within the drinking water and/or feed, the treated feed or water should be shielded from extended exposure to light (e.g., feed should be stored in the dark).

Any natamycin which is consumed by the poultry is normally not absorbed by the poultry. As a result, the natamycin is typically discharged from the poultry in the feces and onto the litter. In an aspect of the invention, the natamycin may be somewhat effective in preventing or reducing the quantity of the undesired yeast which may be present in the litter(e.g., the risk of the poultry being infected by air-borne yeast may be reduced). More importantly, the natamycin is generally not incorporated into the eggs and/or flesh of the poultry and, accordingly, does not affect human consumption of any treated poultry. However, poultry which are exposed to the undesired yeast may incorporate the yeast, and in some cases toxins thereof, into their eggs and flesh. These yeast may be harmful to humans which consume the eggs and poultry flesh, and accordingly, it is desirable to prevent and treat any significant yeast exposure of the poultry. Therefore, the present invention permits enhanced production of poultry which are not contaminated with undesired yeast, toxins thereof and anti-yeast agents.

Although a few aspects and embodiments of the invention have been described in detail, those skilled in the art will readily appreciate that the present invention embraces many combinations, equivalents and variations other than those exemplified.

## Claims

1. Use of natamycin in an effective amount in the manufacture of a composition for the treatment of and/or prophylactic control of diseases in poultry associated with a yeast comprising candida albicans.

2. Use according to claim 1 wherein the poultry are newly hatched chicks.

3. Use according to claim 1 or 2 wherein the effective amount of natamycin comprises from 1 to 70 parts per million of natamycin by weight in a carrier.

4. Use according to claim 3 wherein the carrier is drinking water.

5. Use according to claim 3 wherein the carrier is poultry feedstuff.

6. Use according to claim 5 wherein the natamycin is applied to components of the poultry feed to form a concentrate and then diluted in the feedstuff.

7. Use of natamycin according to any one of the preceding claims to treat crop mycosis.

8. Use according to any one of the preceding claims wherein the poultry are broiler chickens, laying hens or turkey poults.

9. Use according to any one of the preceding claims for treating poultry for an infection of a yeast comprising candida albicans.

10. A poultry feed comprising a cereal grain and natamycin.

11. A feed according to claim 10 wherein the feed also includes an energy source.

12. The method for controlling diseases in poultry associated with a yeast comprising candida albicans comprising:
causing the poultry to consume an effective amount of natamycin and;
continuing to cause the poultry to ingest natamycin for a period which is sufficient to substantially eliminate the growth of the candida albicans.
